(19)

Europäisches
Patentamt
European
Patent Office
Office européen
des brevets

(11) **EP 2 518 474 A1**

(12) **EUROPEAN PATENT APPLICATION**

(43) Date of publication:
**31.10.2012 Bulletin 2012/44**

(51) Int Cl.:
**G01N 21/21** (2006.01)     **G01N 21/27** (2006.01)
**G01N 21/31** (2006.01)

(21) Application number: **11163809.4**

(22) Date of filing: **27.04.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Well Dynamics Applications S.r.l. WDA 42123 Reggio Emilia (IT)**

(72) Inventor: **Ferri, Augusto**
**42122, Reggio Emilia (IT)**

(74) Representative: **Gerli, Paolo**
**Botti & Ferrari S.r.l.**
**Via Cappellini, 11**
**20124 Milano (IT)**

(54) **Method for the determination of analytes in the hair**

(57) The present invention relates to a method to determine the chemical composition of the hair, with special reference to possible analytes of interest like metals, vitamins, drugs, etc. The method generally requires irradiating the hair with polarized light, obtaining a digital pixel-based image thereof, constructing a pixel-based pattern of said image, and elaborating the pattern according to a method further herein described. The invention allows an accurate, reproducible and sensible determination of a large number of analytes in the hair, providing useful information of the physiological/pathological status of the patient, in addition to the quality of the hair itself.

EP 2 518 474 A1

## Description

FIELD OF INVENTION

The present invention relates to the field of hair analysis. A new method of hair analysis is described, based on the interaction of polarized light with analytes present in a hair sample.

BACKGROUND OF INVENTION

Hair analysis has been used from many years to assess human systemic level of metal elements. Hair is widely accepted for assessing toxic elements exposure and level of essential metals, despite the correlation with diseases, metabolic disorders and nutritional status is still object of extensive investigations.

Compared to other types of clinical specimens, hair has different use and even advantages over blood, urine and saliva. While the latter fluids tend to show current or recent body status, hair represent a longer timeframe strictly related to the long term metabolism (Watts D., Trace Elements and Other Essential Nutrients: Clinical application of tissue mineral analysis, InterClinical Laboratories Educational Publications, 2003; Klevay L.M. et al., Am. J. Clin. Nutr., 46, 233-236, 1987).

The same considerations are also valid in veterinary, where mineral concentration in hair is used to assess the mineral status in the animal organism (Underwood E.J. and Suttle N.F., The Mineral Nutrition of Lifestock, 3rd Ed., CAB International, 1999; Combs D.K. et al., J. Anim. Sci., 54, 391-38, 1982).

Hair mineral analysis is routinely carried out by means of a tissue mineral analysis, or thermo-mineralogram analysis (TMA).

The sampled hair is obtained by cutting the first 2-3 centimeters of growth closest to the scalp at the occiput (the nape of the neck) or latero-parietal area (the temples and the back of the head). The sample is washed and then mineralized to completely destroy the organic matrix while conserving the mineral content of the hair by means of a oxidizing acid solution. The aqueous acid solution obtained from mineralization is analyzed using a spectrophotometer in plasma emission at a temperatures ranging from 8.000 to 10.000°C.

However, independent investigations have proven that TMA is not a reliable test by evaluating intra and inter-laboratory results (Schamberger R.J., Biological Trace Element Research, 87, 1-28, 2002; Drasch G. and Roider G., Journal of Trace Elements in Medicine and Biology, 16, 27-31, 2002; Hamilton T. and Schweinsberg F., Versicherungsmedizin, 56, 136-140, 2004; Seidel S. et al., JAMA, 285, 67-72, 2001).

TMA is generally not usable for individual diagnostic with few exceptions because of the large number of factors of individual and environmental influences and sources of error of the method of analysis.

Moreover, TMA cannot detect organic thermo-volatile or thermo-labile compounds, such as amino-acids, vitamins, hormones, etc., which are present in the hair and have an essential role in the metabolisms and nutritionals.

Alternative methods have been proposed to overcome the limitations given by TMA.

US 7,688,943 teaches a method to determine the elemental concentrations in head and body hair by x-ray fluorescence analysis using synchrotron radiation. The metal (calcium) concentration is evaluated by the relative peak heights from fluorescent spectra of the element and background. This method is useful for cancer detection and protection as well as for diagnosing the calcium metabolism.

KR100372526 deals with a method for simultaneous analysis of multiple elements of hair sample by inductively coupled plasma mass spectrometry. The method includes the steps of washing a hair sample with surfactant, deionized water, and acetone, preparing a sample solution by radiating microwave to a nitric acid diluted solution receiving the washed hair sample, grouping the sample solution per detecting element by diluting the sample solution into two groups of 200-300 times (part per billion level) and 2000-30000 times (part per million level), and simultaneously analyzing the multiple elements contained in the hair sample by carrying out the inductively coupled plasma mass spectrometry for the diluted sample solutions.

US 7,625,708 and US 7,629,129 provide a method of using hair follicle bulbs as bio-dosimeters for the detection of chemical exposure. The methods utilize intact, plucked hair follicle bulbs to monitor real-time or near real-time changes in the levels of specific follicular bulb biomarkers to determine exposure to toxicants. By utilizing the living, responsive cells in the plucked hair follicle bulb in an immunohistochemical analysis, the various embodiments mitigate the risks of false positives associated with segmental hair analysis and avoid the more invasive collection required for serum and urine analysis.

US Application 20100002224 describes a device for remotely sensing the presence of hard keratin by means of near and short wavelength infrared light and a microprocessor with algorithms to determine the presence of hard keratin based upon the identification of its spectral characteristic.

However, these attempts continue to show limitations in using of expensive, sophisticated instruments (inductively coupled plasma mass spectrometry, x-ray fluorescence synchrotron radiation) or complex procedures and bonded to single or few elements per analysis (immunohistochemical analysis).

There is still a need for an analytical method to detect trace elements, both thermo-resistive (minerals) and thermo-labile (organic oligo-elements) in hair by means of conventional instruments and simple procedures.

Polarization is principally of importance in chemistry due to circular dichroism and "optical rotation" (circular birefringence) exhibited by optically active (chiral) molecules. It is then used to qualitatively and quantitatively determine the presence of enantiomers, or chiral isomers, and to detect, in general, physical properties related to the presence of chiral centers, mainly in polymer science. In mineralogy, this property is frequently exploited using polarization microscopes, for the purpose of identifying minerals.

Polarized light is also used in hair analysis for determine the morphology and, thus, the physiological conditions of the hair.

US Application 20100105102 describes a method and device to analyze an electronic image of skin or hair samples. The invention relates in general to the field of surface analysis, and more particularly, to a novel system, method and apparatus, for identifying biological markers to analyze skin and hair by means of a mixture of agents which are responsive to light (phosphorescence), pressure or change color (chemi-luminescent) by wetting, pH or chemical reaction and thus provide measurement of transmitted and reflected light.

RU2325109 provides a method for registration of the latent internal damages of hair. It describes a microscopic examination of hair in polarized light where the maximum display of hair in a dark field is reached, blackout and cleared sites on a homogeneous background of the intact hair shaft is tapped on, they are registered and, by that, area of internal deformation of hair is defined.

EP0440907 proposes an apparatus for carrying out hair morphological analyses, in which optical and chemico-physical characteristic data of the hair structure and hair care measures carried out are stored, evaluated and displayed by means of a computer.

Remote sensing is the stand-off collection of information of an object by mean of the electromagnetic radiation (emitted, reflected or refracted) through the use of a variety of devices which are not in physical or intimate contact with the same object. Remote sensing works on the principle of the inverse problem. While the object of interest may not be directly measured, there exists some other variable that can be detected and measured, which may be related to the object of interest through the use of a data-derived computer model.

The term generally refers to the use of imaging sensor technologies, including instruments found in aircraft/ spacecraft and in medical imaging. Earth, planets and stars observation (remote sensing is largely utilized by space agencies such as NASA and ESA), Magnetic Resonance Imaging (MRI) and Positron Emission Tomography (PET) are some examples of remote sensing.

In mathematics, deconvolution or unmixing is an algorithm-based process used to reverse the effects of convolution on recorded data. The concept of deconvolution is widely used in the techniques of signal processing and image processing. These techniques are in turn widely used in many scientific and engineering disciplines.

In general, the object of deconvolution (unmixing) is to find the solution of a convolution equation of the form:

$$y(\lambda) = \sum_{k=1}^{m} a_k e_k(\lambda) + n(\lambda)$$

where $y(\lambda)$ represents the recorded signal and is a linear mixing of $e_k(\lambda)$, reference function of the k pure elements or end members, and $a_k$, relative positive weight of each pure elements, the sum of such relative positive weights being equal to one.

Unmixing is the determination of the $a_k$ weights which minimize the E function by means of the ordinary least square statistical method as

$$E = \sum_{i=1}^{c} \left| y(\lambda_i) - \sum_{k=1}^{m} \alpha_k e_k(\lambda_i) \right|^2$$

knowing the reference function of each pure element $e_k(\lambda)$.

When the reference function is a wavelength spectrum (spectral pattern), hyper-spectral data are used to determine what elements or materials are present in a scene by means of the unmixing method.

The state of the art also teaches that, when a high number of elements are detected and compose the unknown convoluted spectrum, only a relatively limited number of elements effectively have a positive $a_k$ weight and provide a contribution to the amplitude of a particular single $\lambda_k$ wavelength. In other words, given k the number of elements contributing to the convoluted spectrum to be detected, only m elements (with m<k) effectively provide a contribution to the amplitude of a particular single $\lambda_k$ wavelength. The contribution of the other k-m elements to the amplitude of that particular $\lambda_k$ wavelength is practically zero.

Therefore, the quantitative determination of the n elements in a convoluted spectrum can be carried out by weighing the relative presence of $m_1$ elements in the $\lambda_1$ wavelength, $m_2$ elements in the $\lambda_2$ wavelength, $m_j$ elements in the $\lambda_j$ wavelength. Then, the relative presence of the n elements is averaged among the $\lambda_j$ wavelengths detected.

Coupling remote sensing and unmixing are largely used in astronomy (i.e. composition of stars, minerals on a planet surface), geology, mineralogy and mining (i.e. presence of minerals or compounds in an area), agricultural (i.e. vegetation, soil components, grade of plants development) and, more in general, specific applications and targets (i.e. roadways mapping, detection of pollutants and hazardous materials).

In medicine, images of mixed colors are analyzed by deconvolution to provide information on body tissue samples and related pathologies.

EP1636752 describes a system for producing images having mixed colors and analyzing the images to provide deconvoluted images in which colors (in general two) are unmixed from photographic pixels. One color may be used simply for improved visibility of all tissue in the slide, while the other color may bind to protein receptor in tissue affected by a particular disease and provides color data of diagnostic significance, commonly immunohistochemical. Three spectral channels (wavelengths) are used to define the reference pattern of the two end members (dyes), whose full spectral patterns in ultraviolet-visible range is well known.

With the same principle, WO2008025006 describes systems and methods for spectral unmixing of in vivo light data (from mouse). The spectral unmixing separates image data according to spectra from multiple internal light sources in an effort to isolate one or more spectrum of interest. The spectral unmixing obtains images with a combination of different and known excitation and emission limits. The spectral unmixing then uses an iterative solution process to separate spectra for the multiple fluorescent light sources, and provides a spectrum and/or a spatial distribution map, composed by a limited number of spectral wavelengths, for at least one of the internal light sources.

Both the above applications utilize the transmitted intensity of a limited number of selected wavelengths in UV-visible spectrum of non polarized light.

The application of the teaches of EP1636752 and WO2008025006 to the determination of oligo-elements in hair (a use not disclosed in these documents), was found unsatisfactory in our experiments: the relevant tests, using a number of discrete channels (wavelengths) for the quantitative determination of each element, yielded results partially inconsistent with what expected the physiological state of the patient according to the medical literature, and a relatively high mean square error arose from the application of the unmixing methodology; moreover, as the number of the compounds analyzed at the same time increased, some of them could not be detected (values equal to zero); furthermore, non-polarized UV-Visible spectral patterns could only be obtained for some organic substances, while no absorption occurred for other organic substances and for pure mineral elements.

In view of the above prior art, the need is felt for a reliable method of analysis of the hair, capable to detect quali- and quantitatively analytes of interest, with high sensibility and reproducibility.


DESCRIPTION OF THE INVENTION


Now we have unexpectedly found that precise and accurate results of the hair analysis are obtained by a new method wherein polarized light is used not just to provide information of the mechanical structure of the hair, but to obtain a quali-quantitative information on the chemical components of the hair, with special reference to analytes of interest like metals, aminoacids, drugs, hormones, vitamins, pollutants, poisons, etc. This result is made possible by applying a fine method of constructing the standard references (end members), analyzing the hair sample and processing the obtained data, as herein described.

In its general outline, the method requires to irradiate with polarized light a number of pure analytes (i.e. those possibly present in the hair) and to construct for each of them a light pattern in a way herein described; then the hair to be analyzed is irradiated in the same way, and its light pattern is constructed accordingly. The constructed light patterns are then compared against each other via a computer-assisted unmixing processing, allowing to determine the relative contribution of each analyte present in the hair sample. Once the relative contribution of the analyte is determined, its absolute amount and/or concentration in the hair sample (ppm, $\mu$g/mg, etc.) is easily calculated in standard way.

The light patterns are constructed from a microscope digital picture of the sample in question (pure analyte or hair to be analyzed) in polarized light; a matrix of points is then constructed from this picture, wherein each point corresponds to a single pixel of the picture, and is defined by the wavelength and absorbance value of said pixel; all pixels of the picture are included in the matrix; the matrix includes all possible wavelengths of the pixels, i.e. the sampling is not limited to discrete selected wavelengths.

As a result of the above, very precise light patterns are obtained for the standard analytes and for the hair sample.

The matrix data are then processed via software-assisted unmixing; the application of the unmixing algorithm determines the relative contribution of each single analyte to the overall digital picture of the hair sample, providing a very precise and reproducible assessment of the analytes in the hair, including those being present in traces.

Step-wise, the method according to the invention, comprises:

a) [calibration phase]: constructing a light pattern for each pure analytes (end member);

b) [measuring phase]: constructing a light pattern of the hair sample under analysis;

c) [determination phase] comparing the patterns obtained in a) against the pattern obtained in b) and determining the relative contribution of each analyte in the hair sample, via software-assisted unmixing processing.

The calibration phase a) needs not be performed every time before analysing a hair sample. It is generally sufficient to perform it once and then use the resulting light patterns of the pure analytes (end members) as references for an unlimited number of hair samples analysis; these will then include steps b) and c) only.

The calibration and measuring phases a) and b) require the same methodology, the difference being in the sample tested, respectively the pure analyte or the hair sample to be analysed. Said methodology requires:

- refracting an incident polarized light beam through the analyte/hair sample;

- making a microscope digital picture of the analyte/hair sample

- determining, per each pixel of said digital picture, its wavelength and absorbance;

- constructing a spectral pattern (matrix of points), each point being characterized by the wavelength and absorbance values of a pixel of said picture.

In order to obtain the digital picture, the pure analytes (or hair samples) are placed on a microscope glass in their elemental status, i.e. not in solution or mixed with any carriers. The microscope is typically provided with source of a polarized light (e.g. a hot wire lamp provided with a polarizing filter), projecting a beam of polarized light onto the observation glass of the microscope through the sample under observation. The polarized light source may also be external to the microscope body. The irradiation with polarized light of the pure analytes / hair sample is performed by means of a standard microscope light, which works in the visible range typically at a wavelength from 350 to 800 nm. A magnification from 5 to 100 times, preferably from 10 to 30 times, is typically used for the observation of the pure analytes and the hair sample. The microscope is also equipped with (or connected to) a digital camera, with a screen capacity of at least seven million pixels. A picture is then taken of the pure analyte / hair sample under observation. The hair is preferably photographed in its bulb area. The wavelength and transmittance intensity are then determined for each pixel of the picture.

Well-known unmixing algorithm has been used in the *Interactive Data Language* (IDL) elaboration from the pictures. The IDL software evaluates the wavelength and absorbance in each pixel and then compares the absorbance of all the pure analytes at that single wavelength value. A corresponding list of analytes in descending order of absorbance is produced accordingly; within this list, only a fraction of analytes, i.e. those with highest absorbance, will be considered for processing by unmixing. In the present invention, the threshold is typically set at about 15% of the total number of analytes under analysis, since the contribution of absorbance from the other analytes is negligible or zero. Thus for example, in a list of 70 analytes, only the first 10 will be considered for the unmixing processing.

Wishing to further increase the precision of the method, the same sample (pure analyte or hair) can be photographed *n* times (e.g. 1 to 15), producing *n* pre-matrixes, whose data are then averaged to construct the actual final matrix.

In step c), the well-known unmixing algorithm is applied via a software-assisted processing, which enables reaching the final result in a reasonable amount of time. In the invention said unmixing is performed with an adaptation, in that it considers the relative weight of only the most absorbing analytes at each detected wavelength. This correlation can be established by comparing the absorbance of all the pure analytes at a single wavelength value: a corresponding list of analytes in descending order of absorbance is produced accordingly; within this list, only a fraction of analytes, i.e. those with highest absorbance, will be considered for processing by unmixing: in the present invention, the threshold is typically set at about 15% of the total number of analytes under analysis: thus for example, in a list of 70 analytes, only the first 10 will be considered for the unmixing processing.

In the diagnostic test, each hair picture is analyzed in its pixel components, i.e. waveleght and absorbance. The IDL software applies then automatically the unmixing elaboration to the pixel, by selecting among all the end-members (pure analytes) those effectively contributing to the absorbance at that wavelength. The automatic elaboration of all the pixels will provide then the contribution of each end-members and, consequently, the quali-quantitative composition of these analytes in the hair.

Although the unmixing processing provides data relevant to the concentration of all analytes sampled in the calibration phase, they need not be displayed as a whole to the user: in fact, depending on the type of analysis, it may be interesting to obtain information on only some analytes (e.g. vitamins, specific drugs, metals, etc.): in this case the software can be programmed to display information relevant to the analytes of interest.

The software may further be adapted, by standard procedures, to store and/or further elaborate the analysed data, so as to build medical databases assisting the doctor in patient diagnosis, or for research purpose.

Wishing to increase the diagnostic relevance of the present method, the latter will be preferably repeated on different hair samples of the patient, preferably from different areas of the scalp, and averaging the results obtained: this will minimize the possible variability of composition among different hair of a patient.

The analytes considered in the present method are the pure elements and organic molecules, which are potentially present, permanently and/or temporarily in the hair. The pure elements must possess the capacity of refracting an incident polarized light beam, thus generating a colorful refraction spectrum.

The pure elements are minerals, also called metals, nutrient or toxic according to their biological function and definition, non metal elements and, more in general, organic molecules, such as, as a not limitative example, amino-acids, vitamins, hormones, doping or psychotropic compounds, poisons, pollutants.

Not limiting examples of nutrient metals are silver, calcium , cobalt, chrome, iron, lithium , magnesium, manganese, molybdenum, cadmium, potassium, selenium, sodium, strontium, copper, tin, vanadium, zinc.

Not limiting examples of toxic metals are aluminum, arsenic, barium, cadmium, mercury, nickel, lead, uranium.

Not limiting examples of no metal elements are phosphorus, fluorine, iodine, silica, sulfur.

Not limiting examples of amino-acids are aspartic acid, glutamic acid, alanine, arginine, cysteine, phenylalanine, glycine, isoleucine, hystidine, leucine, lysine, methionine, proline, serine, taurine, tyrosine, threonine, tryptophan, valine.

Not limiting examples of vitamins are folic acid, niacin, vitamin A, vitamin B1, vitamin B2, vitamin B5 or pantothenic acid, vitamin B6, vitamin B12, vitamin C, vitamin D, vitamin E, vitamin H or biotin, vitamin K.

Not limiting examples of hormones are estrogen, progesterone, oxytocin, dopamine, serotonin, noradrenalin, adrenalin, testosterone.

Not limiting examples of doping and psychotropic drugs are lysergic acid, cocaine, heroin, morphine, opium extracts and derivatives, cannabis extracts and derivatives, temazepam, lorazepam, and, more in general, benzodiazepine drugs, synthetic drugs.

A further object of the present invention is a method to construct a light pattern of a pure analyte from a microscope digital picture thereof in polarized light, the pattern consisting in a matrix of points, each point defining the wavelength and absorbance of one pixel of the picture; the pattern is constructed by considering all possible wavelengths emitted from the pixels, with no limitation to specific ones.

The invention further extends to a light pattern of a pure analyte, consisting in a matrix of points, each point defining the wavelength and absorbance a pixel of a microscope digital picture of said analyte, the light pattern including all pixels of said digital picture.

The invention also includes the use of one or more analyte light patterns described above, in the chemical quali- and/or quantitative analysis of components of hair.

As documented in the experimental section, the method of the present invention allows to simultaneously detect a very large number of analytes in the hair, including those (cf. minerals) not detectable using the conventional light reflectance-based methods of the prior art; the method proved accurate and reproducible, involving a very low mean standard error; furthermore it proved capable to detect elements even in traces, not otherwise found by said prior art methods.

The invention is now described by reference to the following nonlimiting examples.

EXPERIMENTALS

Example 1.

A sample of 70 elements and compounds has been selected. They include:

- 31 pure elements (minerals), such as silver, calcium, cobalt, chrome, iron, lithium, magnesium, manganese, molybdenum, cadmium, potassium, selenium, sodium, strontium, copper, tin, vanadium, zinc, aluminum, arsenic, barium, cadmium, mercury, nickel, lead, uranium, phosphorus, fluorine, iodine, silica, and sulfur;

- 13 vitamins, such as folic acid, niacin, vitamin A, vitamin B1, vitamin B2, vitamin B5, vitamin B6, vitamin B12, vitamin C, vitamin D, vitamin E, vitamin H, and vitamin K;

- 19 amino-acids, such as aspartic acid, glutamic acid, alanine, arginine, cysteine, phenylalanine, glycine, isoleucine, hystidine, leucine, lysine, methionine, proline, serine, taurine, tyrosine, threonine, tryptophan, and valine;

- 7 hormones, such as estrogen, progesterone, oxytocin, dopamine, serotonin, noradrenalin, and testosterone.

Each elements are poured on a microscope glass and placed in a microscope (Olympus BH2) equipped with a polarizing filter (Nikon). Eight digital pictures of refracted polarized light by the elements are made for each element using a digital camera with at least seven million pixels (Nikon D70S).

The wavelength of the reflected polarized light and the relative transmittance component are determined in each pixel and used to construct the spectral matrix reference patterns (end members) as a mean of the eight pictures.

Example 2

Five vitamins, vitamin A, vitamin B1, vitamin B2, vitamin C and vitamin D, are analyzed.

One single hair is then taken from the latero-parietal area of the head, poured onto a glass in the microscope with polarized light and photographed. The relative amount of the five vitamins in the hair is then evaluated by means of the unmixing algorithm-based process (ordinary least square) having as spectral pattern references (end members) those obtained by a. or b. methods.

a. The UV-visible spectral patterns of the five vitamins are taken as a reference according to the state of the art. Then, six wavelengths, namely red ($\lambda$=700 nm), orange ($\lambda$=620 nm), yellow ($\lambda$=580 nm), green ($\lambda$=530 nm), blue ($\lambda$=470 nm) and violet ($\lambda$=420 nm), are selected and the transmittance component of the six selected wavelengths in each pixel is determined.

b. All the spectral patterns of the five vitamins are taken from Example 1. Then the wavelength of the reflected polarized light and the relative transmittance component are determined in each pixel.

The results are reported in Table 1 and show relatively comparable quantitative outcomes but a much higher statistical significance using the end members obtained as in b.

*Table 1. Concentration of the five vitamins in the analyzed hair and mean square error (MSE) using reference end members as in a. and b.*

| Vitamin | Concentration (a.) | Concentration (b.) |
|---|---|---|
| A | 311,11 ($\mu$g/g) | 193,66 ($\mu$g/g) |
| B1 | 8,04 ($\mu$g/g) | 6,75 ($\mu$g/g) |
| B2 | 10,96 ($\mu$g/g) | 18,20 ($\mu$g/g) |
| C | 5,50 ($\mu$g/g) | 11,58 ($\mu$g/g) |
| D | 47,61 ($\mu$g/g) | 39,77 ($\mu$g/g) |
| MSE | 147,512 | 6,888 |

Example 3

Six amino-acids, arginine (Arg), cysteine (Cys), glycine (Gly), leucine (Leu), proline (Pro) and valine (Val), are analyzed.

One single hair is then taken from the latero-parietal area of the head, poured onto a glass in the microscope with polarized light and photographed. The relative amount of the six amino-acids in the hair is then evaluated by means of the unmixing algorithm-based process (ordinary least square) having as spectral pattern references (end members) those obtained by a. or b. methods.

a. The UV-visible spectral patterns of the six amino-acids are taken as a reference according to the state of the art. Then, six wavelengths, namely red ($\lambda$=700 nm), orange ($\lambda$=620 nm), yellow ($\lambda$=580 nm), green ($\lambda$=530 nm), blue ($\lambda$=470 nm) and violet ($\lambda$=420 nm), are selected and the transmittance component of the six selected wavelengths in each pixel is determined.

b. All the spectral patterns of the six amino-acids are taken from Example 1. Then the wavelength of the reflected polarized light and the relative transmittance component are determined in each pixel.

The results are reported in Table 2 and show relatively comparable quantitative outcomes but a much higher statistical significance using the end members obtained as in b.

*Table 2. Concentration of the six amino-acids in the analyzed hair and mean square error (MSE) using reference end members as in a. and b.*

| Amino-acid | Concentration (a.) | Concentration (b.) |
|---|---|---|
| Arg | 33,23 ($\mu$g/g) | 41,12 ($\mu$g/g) |
| Cys | 74,98 ($\mu$g/g) | 80,10 ($\mu$g/g) |
| Gly | 11,77 ($\mu$g/g) | 9,46 ($\mu$g/g) |
| Leu | 40,95 ($\mu$g/g) | 42,92 ($\mu$g/g) |
| Pro | 28,64 ($\mu$g/g) | 29,49 ($\mu$g/g) |
| Val | 69,57 ($\mu$g/g) | 58,71 ($\mu$g/g) |
| MSE | 197,003 | 8,117 |

Example 4

Three hormones, progesterone, oxytocin and testosterone, are analyzed.

One single hair is then taken from the latero-parietal area of the head, poured onto a glass in the microscope with polarized light and photographed. The relative amount of the three hormones in the hair is then evaluated by means of the unmixing algorithm-based process (ordinary least square) having as spectral pattern references (end members) those obtained by a. or b. methods.

a. The UV-visible spectral patterns of the three hormones are taken as a reference according to the state of the art. Then, six wavelengths, namely red ($\lambda$=700 nm), orange ($\lambda$=620 nm), yellow ($\lambda$=580 nm), green ($\lambda$=530 nm), blue ($\lambda$=470 nm) and violet ($\lambda$=420 nm), are selected and the transmittance component of the six selected wavelengths in each pixel is determined.

b. All the spectral patterns of the three hormones are taken from Example 1. Then the wavelength of the reflected polarized light and the relative transmittance component are determined in each pixel.

The results are reported in Table 3 and show relatively comparable quantitative outcomes but a much higher statistical significance using the end members obtained as in b.

*Table 3. Concentration of the three hormones in the analyzed hair and mean square error (MSE) using reference end members as in a. and b.*

| Hormones | Concentration (a.) | Concentration (b.) |
|---|---|---|
| Progesterone | 15,98 ($\mu$g/g) | 12,49 ($\mu$g/g) |
| Oxytocin | 517,90 ($\mu$g/g) | 480,87 ($\mu$g/g) |
| Testosterone | 173,11 ($\mu$g/g) | 144,64 ($\mu$g/g) |
| MSE | 95,415 | 3,996 |

Example 5.

All the elements and compounds in Example 1 are analyzed.

One single hair is then taken from the latero-parietal area of the head, poured onto a glass in the microscope with polarized light and photographed.

While the relative amount of the elements in the hair is evaluated by means of the unmixing algorithm-based process (ordinary least square) having as spectral pattern references (end members) those obtained by a. or b. methods, the frequency of zero values (no detection of one or more elements) is determined.

a. The UV-visible spectral patterns of the elements are taken as a reference according to the state of the art. Then, six wavelengths, namely red ($\lambda$=700 nm), orange ($\lambda$=620 nm), yellow ($\lambda$=580 nm), green ($\lambda$=530 nm), blue ($\lambda$=470 nm) and violet ($\lambda$=420 nm), are selected and the transmittance component of the six selected wavelengths in each pixel is determined.

b. All the spectral patterns of the compounds are taken from Example 1. Then the wavelength of the reflected polarized light and the relative transmittance component are determined in each pixel.

No zero values have been found in the inventive method, while zero values (no detection of elements) is found according to the state of the art.

Table 4. Frequency of zero values for elements according to the methods a (state of the art) and b (invention).

| Method | a | b |
|---|---|---|
| Zero value frequency | 15/70 | 0/70 |

Example 6

Software (unmixing) accuracy.
One single hair is then taken from the latero-parietal area of the head, poured onto a glass in the microscope with polarized light and photographed.
The picture is examined six time according to our invention in order to check the repeatability and accuracy of the unmixing software.
The results, reported in Table 5 and expressed as raw data in %mg (1 %mg=10 ppm) showed a perfect repeatability of the calculation by unmixing software.

Table 5.

| Element | Run 1 | Run 2 | Run 3 | Run 4 | Run 5 | Run 6 |
|---|---|---|---|---|---|---|
| Aluminum | 4,1328 | 4,1328 | 4,1328 | 4,1328 | 4,1328 | 4,1328 |
| Arsenic | 0,0976 | 0,0976 | 0,0976 | 0,0976 | 0,0976 | 0,0976 |
| Barium | 0,8031 | 0,8031 | 0,8031 | 0,8031 | 0,8031 | 0,8031 |
| Cadmium | 0,6087 | 0,6087 | 0,6087 | 0,6087 | 0,6087 | 0,6087 |
| Mercury | 0,1343 | 0,1343 | 0,1343 | 0,1343 | 0,1343 | 0,1343 |
| Nickel | 0,4790 | 0,4790 | 0,4790 | 0,4790 | 0,4790 | 0,4790 |
| Lead | 2,2303 | 2,2303 | 2,2303 | 2,2303 | 2,2303 | 2,2303 |
| Uranium | 0,0482 | 0,0482 | 0,0482 | 0,0482 | 0,0482 | 0,0482 |
| Silver | 0,0170 | 0,0170 | 0,0170 | 0,0170 | 0,0170 | 0,0170 |
| Calcium | 104,8779 | 104,8779 | 104,8779 | 104,8779 | 104,8779 | 104,8779 |
| Cobalt | 0,2397 | 0,2397 | 0,2397 | 0,2397 | 0,2397 | 0,2397 |
| Chrome | 0,0219 | 0,0219 | 0,0219 | 0,0219 | 0,0219 | 0,0219 |
| Iron | 1,9323 | 1,9323 | 1,9323 | 1,9323 | 1,9323 | 1,9323 |
| Fluorine | 6,0405 | 6,0405 | 6,0405 | 6,0405 | 6,0405 | 6,0405 |
| Phosphorus | 8,7704 | 8,7704 | 8,7704 | 8,7704 | 8,7704 | 8,7704 |
| Iodine | 1,2939 | 1,2939 | 1,2939 | 1,2939 | 1,2939 | 1,2939 |
| Lithium | 0,0160 | 0,0160 | 0,0160 | 0,0160 | 0,0160 | 0,0160 |
| Magnesium | 10,0097 | 10,0097 | 10,0097 | 10,0097 | 10,0097 | 10,0097 |
| Manganese | 0,4462 | 0,4462 | 0,4462 | 0,4462 | 0,4462 | 0,4462 |
| Molybdenum | 0,2042 | 0,2042 | 0,2042 | 0,2042 | 0,2042 | 0,2042 |
| Gold | 0,0561 | 0,0561 | 0,0561 | 0,0561 | 0,0561 | 0,0561 |
| Potassium | 11,7572 | 11,7572 | 11,7572 | 11,7572 | 11,7572 | 11,7572 |

(continued)

| Element | Run 1 | Run 2 | Run 3 | Run 4 | Run 5 | Run 6 |
|---|---|---|---|---|---|---|
| Selenium | 4,4764 | 4,4764 | 4,4764 | 4,4764 | 4,4764 | 4,4764 |
| Silicon | 3,6750 | 3,6750 | 3,6750 | 3,6750 | 3,6750 | 3,6750 |
| Sodium | 16,9296 | 16,9296 | 16,9296 | 16,9296 | 16,9296 | 16,9296 |
| Tin | 0,1877 | 0,1877 | 0,1877 | 0,1877 | 0,1877 | 0,1877 |
| Strontium | 2,1767 | 2,1767 | 2,1767 | 2,1767 | 2,1767 | 2,1767 |
| Copper | 13,0530 | 13,0530 | 13,0530 | 13,0530 | 13,0530 | 13,0530 |
| Vanadium | 0,1406 | 0,1406 | 0,1406 | 0,1406 | 0,1406 | 0,1406 |
| Zinc | 16,6080 | 16,6080 | 16,6080 | 16,6080 | 16,6080 | 16,6080 |
| Sulfur | 29588,2734 | 29588,2734 | 29588,2734 | 29588,2734 | 29588,2734 | 29588,2734 |
| Folic Acid | 7,1941 | 7,1941 | 7,1941 | 7,1941 | 7,1941 | 7,1941 |
| Niacin | 0,0000 | 0,0000 | 0,0000 | 0,0000 | 0,0000 | 0,0000 |
| Vitamin A | 19,5961 | 19,5961 | 19,5961 | 19,5961 | 19,5961 | 19,5961 |
| Vitamin B1 | 1,6515 | 1,6515 | 1,6515 | 1,6515 | 1,6515 | 1,6515 |
| Vitamin B2 | 1,8069 | 1,8069 | 1,8069 | 1,8069 | 1,8069 | 1,8069 |
| Vitamin B5 | 2,0492 | 2,0492 | 2,0492 | 2,0492 | 2,0492 | 2,0492 |
| Vitamin B6 | 4,7349 | 4,7349 | 4,7349 | 4,7349 | 4,7349 | 4,7349 |
| Vit. B12 - Pant. Ac. | 0,8868 | 0,8868 | 0,8868 | 0,8868 | 0,8868 | 0,8868 |
| Vitamin C | 2,3177 | 2,3177 | 2,3177 | 2,3177 | 2,3177 | 2,3177 |
| Vitamin D | 9,3624 | 9,3624 | 9,3624 | 9,3624 | 9,3624 | 9,3624 |
| Vitamin E | 2,6536 | 2,6536 | 2,6536 | 2,6536 | 2,6536 | 2,6536 |
| Vitamin H-Biotin | 3,6945 | 3,6945 | 3,6945 | 3,6945 | 3,6945 | 3,6945 |
| Vitamin K | 1,0806 | 1,0806 | 1,0806 | 1,0806 | 1,0806 | 1,0806 |
| Aspartic Acid | 3,2327 | 3,2327 | 3,2327 | 3,2327 | 3,2327 | 3,2327 |
| Glutamic Acid | 17,4440 | 17,4440 | 17,4440 | 17,4440 | 17,4440 | 17,4440 |
| Alanine | 1,3570 | 1,3570 | 1,3570 | 1,3570 | 1,3570 | 1,3570 |
| Arginine | 4,1969 | 4,1969 | 4,1969 | 4,1969 | 4,1969 | 4,1969 |
| Cysteine | 6,8337 | 6,8337 | 6,8337 | 6,8337 | 6,8337 | 6,8337 |
| Phenylalanine | 1,6679 | 1,6679 | 1,6679 | 1,6679 | 1,6679 | 1,6679 |
| Glycine | 2,8222 | 2,8222 | 2,8222 | 2,8222 | 2,8222 | 2,8222 |
| Isoleucine | 2,6210 | 2,6210 | 2,6210 | 2,6210 | 2,6210 | 2,6210 |
| Hystidine | 4,6919 | 4,6919 | 4,6919 | 4,6919 | 4,6919 | 4,6919 |
| Leucine | 4,3258 | 4,3258 | 4,3258 | 4,3258 | 4,3258 | 4,3258 |
| Lysine | 1,5562 | 1,5562 | 1,5562 | 1,5562 | 1,5562 | 1,5562 |
| Methionine | 3,0377 | 3,0377 | 3,0377 | 3,0377 | 3,0377 | 3,0377 |
| Proline | 3,3247 | 3,3247 | 3,3247 | 3,3247 | 3,3247 | 3,3247 |
| Serine | 6,9733 | 6,9733 | 6,9733 | 6,9733 | 6,9733 | 6,9733 |
| Taurine | 3,1625 | 3,1625 | 3,1625 | 3,1625 | 3,1625 | 3,1625 |
| Tyrosine | 2,7557 | 2,7557 | 2,7557 | 2,7557 | 2,7557 | 2,7557 |

(continued)

| Element | Run 1 | Run 2 | Run 3 | Run 4 | Run 5 | Run 6 |
|---|---|---|---|---|---|---|
| Threonine | 6,0334 | 6,0334 | 6,0334 | 6,0334 | 6,0334 | 6,0334 |
| Tryptophan | 3,4695 | 3,4695 | 3,4695 | 3,4695 | 3,4695 | 3,4695 |
| Valine | 8,4102 | 8,4102 | 8,4102 | 8,4102 | 8,4102 | 8,4102 |
| Estrogen | 2,0757 | 2,0757 | 2,0757 | 2,0757 | 2,0757 | 2,0757 |
| Progesterone | 1,0565 | 1,0565 | 1,0565 | 1,0565 | 1,0565 | 1,0565 |
| Ossitocina | 43,3445 | 43,3445 | 43,3445 | 43,3445 | 43,3445 | 43,3445 |
| Dopamina | 2,1654 | 2,1654 | 2,1654 | 2,1654 | 2,1654 | 2,1654 |
| Serotonina | 13,2058 | 13,2058 | 13,2058 | 13,2058 | 13,2058 | 13,2058 |
| Noradrenalina | 0,8484 | 0,8484 | 0,8484 | 0,8484 | 0,8484 | 0,8484 |
| Testosterone | 12,7456 | 12,7456 | 12,7456 | 12,7456 | 12,7456 | 12,7456 |

Example 7

Accuracy of the method according to the invention.

One single hair is then taken from the latero-parietal area of the head, poured onto a glass in the microscope with polarized light and photographed six times. Each picture is then elaborated according to the invention in order to calculate the concentrations of the elements and compounds in the hair and the relative standard deviation (sd) for each element and compound.

As reported in Table 6, the standard deviation for each element is low, meaning the very good accuracy of the analytical method.

Table 6. Concentration and standard deviation (sd) of the elements and compounds in the analyzed hair. The concentrations are expressed in $\mu g/g$, or ppm (part per million), with the exception of calcium and sulfur, whose values are in mg/g (also evidenced by *).

| Element | ppm ($\mu g/g$) | sd | Element | ppm ($\mu g/g$) | sd |
|---|---|---|---|---|---|
| Aluminum | 54,91 | 0,77 | Serotonin | 170,19 | 2,40 |
| Arsenic | 1,01 | 0,00 | Noradrenalin | 9,38 | 0,06 |
| Barium | 9,81 | 0,03 | Testosterone | 176,38 | 0,85 |
| Cadmium | 13,42 | 0,32 | Folic Acid | 140,77 | 1,39 |
| Mercury | 0,95 | 0,00 | Niacin | 0,00 | 0,00 |
| Nickel | 5,89 | 0,05 | Vitamin A | 235,75 | 2,30 |
| Lead | 28,58 | 0,02 | Vitamin B1 | 27,18 | 0,69 |
| Uranium | 0,93 | 0,03 | Vitamin B2 | 24,91 | 0,54 |
| Silver | 0,25 | 0,01 | Vitamin B5 | 14,95 | 0,05 |
| Calcium * (in mg/g) | 1,43 | 0,03 | Vitamin B6 | 61,58 | 1,15 |
| Cobalt | 3,03 | 0,07 | Vitamin B12 - Pant. | 15,73 | 0,04 |
| | | | Ac. | | |
| Chrome | 0,35 | 0,00 | Vitamin C | 34,64 | 0,73 |
| Iron | 18,31 | 0,39 | Vitamin D | 136,32 | 2,45 |
| Fluorine | 31,13 | 0,58 | Vitamin E | 36,70 | 0,05 |
| Phosphorus | 70,73 | 0,59 | Vitamin H- Biotin | 25,03 | 0,10 |

(continued)

| Element | ppm (μg/g) | sd | Element | ppm (μg/g) | sd |
|---|---|---|---|---|---|
| Iodine | 16,61 | 0,05 | Vitamin K | 15,25 | 0,04 |
| Lithium | 0,19 | 0,02 | Aspartic Acid | 20,44 | 0,05 |
| Magnesium | 135,63 | 9,04 | Glutamic Acid | 248,08 | 0,34 |
| Manganese | 5,03 | 0,16 | Alanine | 15,53 | 0,01 |
| Molybdenum | 2,57 | 0,08 | Arginine | 61,92 | 0,01 |
| Gold | 0,94 | 0,03 | Cysteine | 73,54 | 0,58 |
| Potassium | 160,40 | 12,23 | Phenylalanine | 22,05 | 0,01 |
| Selenium | 58,54 | 0,28 | Glycine | 28,99 | 0,11 |
| Silicon | 47,56 | 1,58 | Isoleucine | 40,95 | 0,13 |
| Sodium | 227,88 | 1,29 | Hystidine | 39,94 | 0,40 |
| Tin | 1,99 | 0,01 | Leucine | 54,48 | 0,26 |
| Strontium | 26,63 | 0,20 | Lysine | 16,23 | 0,13 |
| Copper | 236,40 | 2,40 | Methionine | 33,70 | 0,12 |
| Vanadium | 0,39 | 0,01 | Proline | 23,91 | 0,05 |
| Zinc | 186,26 | 0,95 | Serine | 85,12 | 0,06 |
| Sulfur * (in mg/g) | 503,7 | 9,6 | Taurine | 50,11 | 0,71 |
| Estrogen | 19,95 | 0,18 | Tyrosine | 36,24 | 0,10 |
| Progesterone | 16,54 | 0,17 | Threonine | 65,62 | 0,04 |
| Oxyitocin | 621,55 | 2,14 | Tryptophan | 43,93 | 0,04 |
| Dopamine | 26,92 | 0,54 | Valine | 141,62 | 0,90 |

**Claims**

1. A method to analyze the composition of a hair sample, based on processing light patterns obtained from a microscope digital picture in polarized light of said hair sample, wherein the amount of each analyte present in the hair sample is calculated via unmixing.

2. The method of claim 1 comprising:

   a) a calibration phase, wherein the light patterns of pure analytes (end members) are constructed;
   b) a measuring phase, wherein the light pattern of the hair sample is constructed;
   c) a determination phase, wherein the light patterns of a) and b) are compared and elaborated via software-assisted unmixing, thereby determining the relative contribution of each analyte present in the hair sample, and therefrom its absolute amount or concentration.

3. The method of claims 1-2, wherein each light pattern consists in a matrix of points equal to the number of pixels contained in the corresponding digital picture, each point defining the wavelength and absorbance of one pixel of the picture.

4. The method of claims 1-3, wherein all patterns are constructed by considering all possible wavelengths emitted from the pixels, i.e. with no limitation to preferred wavelengths.

5. The method of claims 2-4, wherein the calibration phase a) is performed once only, and the obtained patterns (end members) are used as a standard for testing different hair samples according to steps b) and c).

6. The method of claims 1-5, wherein said light patterns are obtained by averaging the data from 2-15 pictures of the same sample.

7. The method of claims 1-6, wherein the unmixing utilizes the relative weight of the most absorbing analytes at each detected wavelength, said most absorbing analytes corresponding to about 15% of the total number of analytes under testing.

8. The method according to claims 1-7, wherein the analytes include one or more among metals, non-metal elements, aminoacids, vitamins, hormones, doping and/or psychotropic drugs, poisons, pollutants.

9. The method according to claim 8, wherein:

   the metals include one or more among: silver, calcium , cobalt, chrome, iron, lithium , magnesium, manganese, molybdenum, cadmium, potassium, selenium, sodium, strontium, copper, tin, vanadium, zinc, aluminum, arsenic, barium, cadmium, mercury, nickel, lead, uranium;
   the non metal elements include one or more among phosphorus, fluorine, iodine, silica, sulfur;
   the amino-acids include one or more among aspartic acid, glutamic acid, alanine, arginine, cysteine, phenylalanine, glycine, isoleucine, hystidine, leucine, lysine, methionine, proline, serine, taurine, tyrosine, threonine, tryptophan, valine;
   the vitamins include one or more among folic acid, niacin, vitamin A, vitamin B1, vitamin B2, vitamin B5 or pantothenic acid, vitamin B6, vitamin B12, vitamin C, vitamin D, vitamin E, vitamin H or biotin, vitamin K;
   the hormones include one or more among estrogen, progesterone, oxytocin, dopamine, serotonin, noradrenalin, adrenalin, testosterone.

10. A method to construct a light pattern of a pure analyte from a microscope digital picture thereof in polarized light, the pattern consisting in a matrix of points, each point defining the wavelength and absorbance of one pixel of the picture.

11. The method of claim 10, wherein said pattern is constructed by considering all possible wavelengths recorded from the pixels, with no limitation to specific wavelengths.

12. A light pattern of a pure analyte (end member) consisting in a matrix of points, each point defining the wavelength and absorbance a pixel of a microscope digital picture of said analyte, the light pattern including all pixels of said digital picture.

13. A light pattern of an analyte according to claim 12, useful as a standard for the determination of said analyte in a hair sample.

14. Use of one or more analyte light patterns according to claim 12, in the chemical quali- and/or quantitative analysis of the hair.

Europäisches
Patentamt

European
Patent Office

Office européen
des brevets

# EUROPEAN SEARCH REPORT

Application Number

EP 11 16 3809

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | D.J.W. Dikken, D. Broekhuizen: "Two-photon autofluorescence spectral imaging of hair samples", Faculty Science, Universiteit Utrecht , 30 January 2006 (2006-01-30), XP002658503, Retrieved from the Internet: URL:http://www.phys.uu.nl/~0426989/Zooi/Two-photon%20autofluorescence%20spectral%20imaging%20of%20hair%20samples.pdf [retrieved on 2011-09-06] * paragraph [experiment] * ----- | 1-14 | INV. G01N21/21 G01N21/27 G01N21/31 |
| X | RAVN C ET AL: "Near-infrared chemical imaging (NIR-CI) on pharmaceutical solid dosage forms-Comparing common calibration approaches", JOURNAL OF PHARMACEUTICAL AND BIOMEDICAL ANALYSIS, NEW YORK, NY, US, vol. 48, no. 3, 4 November 2008 (2008-11-04), pages 554-561, XP025479158, ISSN: 0731-7085, DOI: 10.1016/J.JPBA.2008.07.019 [retrieved on 2008-07-31] * paragraph [2.4.3.2] * ----- | 12 | |
| L | Anonymous: "index of /~0426989/Zooi", apache/2.2.3 Server at Faculty Science, Universiteit Utrecht port 80 , XP002658504, Retrieved from the Internet: URL:http://www.phys.uu.nl/~0426989/Zooi/ [retrieved on 2011-09-06] * the whole document * ----- | 1-14 | TECHNICAL FIELDS SEARCHED (IPC) G01N G01J A61B G02B |

-/--

The present search report has been drawn up for all claims

| Place of search | Date of completion of the search | Examiner |
|---|---|---|
| The Hague | 8 September 2011 | Rasmusson, Marcus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 11 16 3809

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 784 162 A (CABIB DARIO [IL] ET AL) 21 July 1998 (1998-07-21) * claims 1-4,34-36,51-53 * | 1-14 | |
| A | KALASINSKY K S ET AL: "Hair analysis by infrared microscopy for drugs of abuse", FORENSIC SCIENCE INTERNATIONAL, vol. 63, no. 1-3, 1 December 1993 (1993-12-01), pages 253-260, XP026456584, ELSEVIER SCIENTIFIC PUBLISHERS IRELAND LTD, IE ISSN: 0379-0738 [retrieved on 1993-12-01] * the whole document * | 1-14 | |
| A | BIRNGRUBER C ET AL: "The color(s) of human hair-Forensic hair analysis with SpectraCube<(>R)", FORENSIC SCIENCE INTERNATIONAL, vol. 185, no. 1-3, 10 March 2009 (2009-03-10), pages E19-E23, XP025959305, ELSEVIER SCIENTIFIC PUBLISHERS IRELAND LTD, IE ISSN: 0379-0738, DOI: 10.1016/J.FORSCIINT.2008.12.018 [retrieved on 2009-01-24] * the whole document * | 1-14 | |

TECHNICAL FIELDS
SEARCHED        (IPC)

The present search report has been drawn up for all claims

| Place of search | Date of completion of the search | Examiner |
|---|---|---|
| The Hague | 8 September 2011 | Rasmusson, Marcus |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

**ANNEX TO THE EUROPEAN SEARCH REPORT
ON EUROPEAN PATENT APPLICATION NO.**

EP 11 16 3809

This annex lists the patent family members relating to the patent documents cited in the above-mentioned European search report.
The members are as contained in the European Patent Office EDP file on
The European Patent Office is in no way liable for these particulars which are merely given for the purpose of information.

08-09-2011

| Patent document cited in search report | | Publication date | Patent family member(s) | Publication date |
|---|---|---|---|---|
| US 5784162 | A | 21-07-1998 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7688943 B
- KR 100372526
- US 7625708 B
- US 7629129 B
- US 20100002224 A
- US 20100105102 A
- RU 2325109
- EP 0440907 A
- EP 1636752 A
- WO 2008025006 A

**Non-patent literature cited in the description**

- **WATTS D.** Trace Elements and Other Essential Nutrients: Clinical application of tissue mineral analysis. *InterClinical Laboratories Educational Publications,* 2003
- **KLEVAY L.M. et al.** *Am. J. Clin. Nutr.,* 1987, vol. 46, 233-236
- The Mineral Nutrition of Lifestock. **UNDERWOOD E.J. ; SUTTLE N.F.** CAB International. 1999
- **COMBS D.K. et al.** *J. Anim. Sci.,* 1982, vol. 54, 391-38
- **SCHAMBERGER R.J.** *Biological Trace Element Research,* 2002, vol. 87, 1-28
- **DRASCH G. ; ROIDER G.** *Journal of Trace Elements in Medicine and Biology,* 2002, vol. 16, 27-31
- **HAMILTON T. ; SCHWEINSBERG F.** *Versicherungsmedizin,* 2004, vol. 56, 136-140
- **SEIDEL S. et al.** *JAMA,* 2001, vol. 285, 67-72